(19) Europäisches Patentamt
European Patent Office
Office européen des brevets

(11) Veröffentlichungsnummer : **0 175 315**
**B1**

(12) # EUROPÄISCHE PATENTSCHRIFT

(45) Veröffentlichungstag der Patentschrift : **11.01.89**

(51) Int. Cl.⁴ : **C 07 F 9/38**, A 61 K 31/66

(21) Anmeldenummer : **85111655.8**

(22) Anmeldetag : **14.09.85**

(54) **4-Dimethylamino-1-hydroxybutan-1,1-diphosphonsäure, deren wasserlösliche Salze, Verfahren zu ihrer Herstellung sowie ihre Verwendung.**

(30) Priorität : **21.09.84 DE 3434667**

(43) Veröffentlichungstag der Anmeldung : **26.03.86 Patentblatt 86/13**

(45) Bekanntmachung des Hinweises auf die Patenterteilung : **11.01.89 Patentblatt 89/02**

(84) Benannte Vertragsstaaten : **AT BE CH DE FR GB IT LI NL SE**

(56) Entgegenhaltungen :
**DE-A- 3 313 049**
**FR-A- 2 259 615**
**FR-A- 2 319 646**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Blum, Helmut**
**Bertha-von-Suttner-Strasse 30**
**D-4000 Düsseldorf 13 (DE)**
Erfinder : **Hemmann, Siglinde**
**Kantstrasse 28**
**D-4005 Meerbusch 1 (DE)**

**EP 0 175 315 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft 4-Dimethyl-amino-1-hydroxybutan-1,1-diphosphonsäure (im Nachstehenden auch abgekürzt DABD genannt), deren wasserlösliche Salze, ein Verfahren zu ihrer Herstellung sowie ihre Verwendung.

Die DE-PS 25 34 391 beschreibt 1-Hydroxy-3-aminoalkan-1,1-diphosphonsäuren, deren Herstellung sowie deren Verwendung als Komplexbildner für Erdalkali-Ionen, vorzugsweise Calciumionen. Es wird auch die pharmazeutische Anwendung dieser Verbindungen beschrieben.

Die DE 24 05 254 beschreibt die Verwendung von 3-Amino-1-hydroxypropan-1,1-diphosphonsäure oder ihrer wasserlöslichen Salze bei der Beeinflussung von Calciumstoffwechselstörungen im menschlichen oder tierischen Körper.

Die europäische Patentschrift 39 033 beschreibt ein verbessertes Verfahren zur Herstellung von ω-Amino-1-hydroxyalkyliden-1,1-bisphosphonsäuren.

Die Aufgabe der vorliegenden Erfindung besteht darin, eine neue Verbindung zu schaffen, die verbesserte komplexbildende Eigenschaften aufweist.

Dementsprechend betrifft die vorliegende Erfindung 4-Dimethylamino-1-hydroxybutan-1,1-diphosphonsäure, der Formel

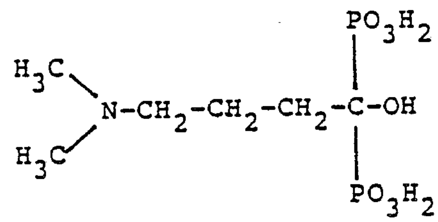

deren Salze sowie Verfahren zu ihrer Herstellung.

Die Erfindung betrifft ferner die Verwendung der 4-Dimethylamino-1-hydroxybutan-1,1-diphosphonsäure bzw. deren wasserlöslichen Salze zum Komplexieren insbesondere von Erdalkali-Ionen, vorzugsweise Calciumionen.

Die Herstellung der DABD erfolgt durch Phosphonylierung von 4-Dimethylaminobuttersäurehydrochlorid mit phosphoriger Säure in Anwesenheit von Phosphorylchlorid. Es ist ebenfalls möglich, die DABD gemäß den bekannten anderen Phosphonylierungsreaktionen (z. B. Verwendung von $PCl_3$ etc.) oder auch nach dem Verfahren des europäischen Patents 39 033 herzustellen.

Als Ausgangsprodukt wird normalerweise 4-Aminobuttersäure eingesetzt, die nach der aus Houben-Weyl 11/2 bekannten Methode über eine reduzierende Methylierung nach Leuckart dialkyliert wird.

Die DABD bzw. deren wasserlösliche Salze besitzen ein ausgezeichnetes Komplexiervermögen gegenüber 2- und mehrwertigen Metallionen, wie beispielsweise gegenüber Ca, Cu, Fe, Cr und anderen, bei verschiedenen Temperaturen und pH-Werten.

Insbesondere zeigen sie ein besonders gutes Calcium-Bindevermögen, das besser ist als dasjenige bekannter analoger Verbindungen, wie sich in einfacher Weise durch den « Hampshire-Test » nachweisen läßt.

Daher sind die DABD sowie deren wasserlösliche Salze geeignet zur Verwendung als Komplexiermittel bzw. Sequestriermittel.

Außer dem ausgezeichneten Komplexiervermögen zeichen sich DABD bzw. deren wasserlösliche Salze durch eine starke Threshold-Aktivität aus, d. h. sie sind in der Lage, die Ausfällung schwerlöslicher Erdalkalimetallsalze auch in Impfmengen, das sind unterstöchiometrische Mengen, zu verhindern.

DABD bzw. deren wasserlösliche Salze sind als Komplexiermittel sehr vielseitig verwendbar: Beispielsweise können sie speziell für die Vorgänge der Wasserenthärtung Anwendung finden, wobei die vorstehend erwähnte Threshold-Wirkung eine wesentliche Rolle spielt. Es ist daher nicht notwendig, mit stöchiometrischen Mengen zu arbeiten, sondern man kann auch mit unterstöchiometrischen Mengen Calcitfällungen erheblich verzögern.

Sie sind auch als Korrosions- und Steinansatzverhütungsmittel für Kühlwässer, insbesondere in Kombination mit an sich bekannten Zusätzen, gut geeignet.

Die genannten Eigenschaften bewirken, daß die DABD bzw. deren wasserlösliche Salze beispielsweise auch für die Entkrustung von Geweben, in denen sich Erdalkalimetallsalze abgelagert haben, und zur Verminderung der Ascheanreicherung in Geweben Anwendung finden können. Sie kommen auch als Buildersubstanzen mit komplexierenden Eigenschaften in Wasch- und Reinigungsmitteln in Frage und können in Kombination mit bekannten anionenaktiven, kationenaktiven oder nichtionogenen Netzmitteln verwendet werden. Weiterhin können sie in Kombination mit Ätzalkalien, Alkalicarbonaten, Alkalisilikaten, Phosphaten oder Boraten Anwendung finden.

Aufgrund ihres starken Komplexierungsvermögens können sie auch mit Vorteil in Systemen eingesetzt werden, in denen Schwermetallionen, beispielsweise Kupferionen, unerwünschte Effekte

auslösen. Beispielhaft seien hier genannt die Zersetzung von Perverbindungen in Bleichmitteln bzw. Verfärbungen oder Ranzigwerden von Fetten und Seifen.

Die DABD bzw. deren wasserlösliche Salze sind ferner geeignet für Reinigungsprozesse von starren Gegenständen wie insbesondere Metall oder Glas. Hierbei kommt insbesondere die Verwendung als Zusatz zu Flaschenspülmitteln in Betracht.

Die erfindungsgemäßen Produkte sind auch geeignet für pharmazeutische Zwecke, insbesondere zur Behandlung von Störungen des Calcium- bzw. des Phosphatstoffwechsels und den damit verbundenen Erkrankungen. Weiterhin können die DABD und deren wasserlösliche Salze in kosmetischen Präparaten, wie insbesondere Zahnpasta, Mundwasser und ähnlichen Produkten, verwendet werden, da sie die Bildung von Zahnstein wesentlich reduzieren bzw. inhibieren.

Geeignete wasserlösliche Salze der DABD sind Natrium-, Kalium-, Ammonium- und substituierte Ammoniumsalze, wie Mono-, Di- oder Triethanolammoniumsalze. Auch Lithiumsalze können Anwendung finden. Bevorzugt sind die Natrium-, Kalium- und Ammoniumsalze sowie Dialkylammoniumsalze und Triethanolaminsalze. Es können auch Mischungen der Salze verwendet werden.

Bei der pharmazeutischen Anwendung ist die Dosierung der verwendeten Verbindungen variabel und hängt von den jeweiligen Konditionen, wie Art und Schwere der Erkrankung, Dauer der Behandlung und der jeweiligen Verbindung ab. Einzelne Dosierungen können von 0,05 bis 500 mg pro kg Körpergewicht und Tag betragen. Die bevorzugte Dosierung beträgt 1 bis 50 mg pro kg Körpergewicht und Tag und kann in bis zu 4 % täglich verabreicht werden. Die höheren Dosierungen sind bei oraler Applikation infolge der begrenzten Resorption erforderlich. Bei länger dauernden Behandlungen sind nach anfänglich höheren Dosierungen normalerweise geringere Dosierungen notwendig, um den gewünschten Effekt aufrechtzuerhalten.

Die DABD bzw. deren Salze können sowohl oral als auch in hypertonischer Lösung subkutan, intramuskulär oder intravenös appliziert werden. Die bevorzugten Dosisbereiche für diese Anwendung sind (in mg/kg · Tag)

| | |
|---|---|
| oral | 1 bis 50 |
| subkutan | 1 bis 50 |
| intramuskulär | 0,05 bis 10 |
| intravenös | 0,05 bis 2 |

Die Substanzen können zur Verabreichung in Tabletten, Pillen, Kapseln oder Injektionslösungen formuliert werden. Für Tiere können die Substanzen auch im Futter bzw. als Futterzusätze Verwendung finden.

Schließlich eignen sich DABD und deren Salze als Zusatz zu Präparaten für die Herstellung von $99^{m}$-Technetium-Radiodiagnostika.

Die Erfindung wird durch die nachstehenden Beispiele und Versuche weiter erläutert.

## Beispiel 1

Unter Inertgasatmosphäre wurde ein Gemisch von 1 Mol Phosphoriger Säure und 1 Mol Phosphorylchlorid hergestellt und dann langsam 0.5 Mol Dimethylaminobuttersäure-hydrochlorid zugegeben. Das Reaktionsgemisch wurde auf 100 °C aufgeheizt und 4 Stdn. bei dieser Temperatur belassen. Während dieser Zeit erstarrte das Reaktionsgut zu einer schaumartigen Masse, die nicht mehr rührbar war. Die Reaktionsmasse wurde nach dem Abkühlen mit 225 ml Wasser versetzt und zwecks vollständiger Hydrolyse aufgekocht. Das Hydrolysat wurde über Aktivkohle blank filtriert und dann mit der gleichen Volumenmenge Methanol versetzt.

Nach mehrstündigem Stehen wurde die ausgefallene kristalline 4-Dimethylamino-1-hydroxybutan-1,1-diphosphonsäure abgefrittet, mit Methanol gewaschen und bei 60 °C getrocknet.

Ausbeute : 64 %.
Smp. : 212 °C
MM lt. pH-Titr. gef. 279.8 (1. Wdp.), 278.3 (2. Wdp.)
ber. 277

Elementaranalyse

| | | | |
|---|---|---|---|
| C 25,8 | H 6,29 | N 4,96 | P 22,2 |
| C (25,99) | H (6,14) | N (5,05) | P (22,38) |

## Beispiel 2

0,1 Mol der beschriebenen Aminodiphosphonsäure wurden in 125 ml Wasser suspendiert und mit der für das Dinatriumsalz berechneten Menge an 10 %iger Natronlauge versetzt. Der pH-Wert der Lösung betrug nach der Neutralisation 8,3. Durch Fällung mit einem Gemisch aus Ethanol/Methanol wurde ein Öl erhalten, das durch weitere Alkoholbehandlung verfestigt wurde. Nach dem Trocknen über Blaugel

wurde ein Salz mit einem Restwassergehalt von ca. 10 % erhalten. Durch Trocknen bei 110 °C über $P_2O_5$ wurde das Dinatrium-4-dimethylamino-1-hydroxybutan-1,1-diphosphonat in wasserfreier Form isoliert.

% C 22,3 (ber. 22,4), % H 4,51 (4,67),
% P 19,1 (19,3), N 4,31 (4,36), Na 14,2 (14,3)

Molmasse gemäß potentiometrischer Titration :
317,1 (ber. 321).

### Vergleichsbeispiel 1

Die komplexbildenden Eigenschaften der DABD wurden mit der bekannten Methode des Hampshire-Tests bestimmt. Ebenfalls wurden die komplexierenden Eigenschaften bekannter ähnlicher Verbindungen zum Vergleich bestimmt. Die Werte sind in der nachstehenden Tabelle I genannt.

Tabelle I

| | Komplexiermittel | mg $CaCO_3$/g Säure, pH 11 |
|---|---|---|
| A | $Me_2N(CH_2)_3C(OH)(PO_3H_2)_2$ | > 5000 |
| B | $H_2N(CH_2)_3C(OH)(PO_3H_2)_2$ | 600 |
| C | $CH_3CH(NH_2)CH_2C(OH)(PO_3H_2)_2$ | 710 |
| D | $H_2N(CH_2)_2C(OH)(PO_3H_2)_2$ | 470 |
| E | $Me_2N(CH_2)_2C(OH)(PO_3H_2)_2$ | > 2500 |

A = gemäß der Erfindung
B — E = Vergleichsverbindungen

### Vergleichsbeispiel 2

Die Threshold-Aktivität, d. h. die Fähigkeit des Komplexbildners, in unterstöchiometrischen Mengen das Scaling schwerlöslicher Calciumsalze (Gips und Calcit) zu verhindern bzw. zu verzögern, wurde in synthetischen Salzsolen, die in ihrer Zusammensetzung Lagerstätten-Injektionswässern niedriger Salinität entsprechen, getestet.

Die Threshold-Aktivität der erfindungsgemäßen Diphosphonsäure wurde bei Gips im Impfbereich von 1 bis 20 ppm, bei Calcit von 5 bis 50 ppm untersucht.

Bei der Gips-Inhibition wurde eine Calciumsalzlösung von 211 °d und bei der Calcit-Inhibition von 282 °d, bestehend aus einer Mischhärte von 231 °d Ca- und 51 °d Mg-Härte, eingesetzt. Die Testlösungen wurden 3 Tage bei 70 °C im Wasserbad gelagert. Anschließend wurde der in Lösung verbliebene Anteil an Erdalkalisalz bestimmt.

Die Bestimmung erfolgte nach der Standard-Testmethode 03-74 der National Association of Corrosion Engineers.

Außerdem wurde zum Vergleich noch die Threshold-Aktivität bekannter ähnlicher Verbindungen bestimmt. Die Ergebnisse sind in den nachstehenden Tabellen II und III aufgeführt.

(Siehe Tabellen II-III Seite 5 ff.)

Tabelle II

Gips-Inhibierung (einges. 5134 mg $CaSO_4$/l)

| | Inhibitor | 1 ppm | 3 ppm | 5 ppm | 10 ppm | 20 ppm | Blindwert |
|---|---|---|---|---|---|---|---|
| | | Verbliebener Calciumsulfatanteil in Lösung in mg/l | | | | | |
| A | $Me_2N(CH_2)_3C(OH)(PO_3H_2)_2$ | 5012 | 5102 | 5090 | 5140 | 5104 | 3473 |
| B | $H_2N(CH_2)_3C(OH)(PO_3H_2)_2$ | 3744 | 3812 | 3800 | 3974 | 4220 | 3460 |
| C | $CH_3CH(NH_2)CH_2C(OH)(PO_3H_2)_2$ | 3400 | 3482 | 3700 | 3824 | 4190 | 3617 |
| D | $H_2N(CH_2)_2C(OH)(PO_3H_2)_2$ | 3498 | 3526 | 3554 | 3800 | 4010 | 3512 |
| E | $Me_2N(CH_2)_2C(OH)(PO_3H_2)_2$ | 3583 | 3738 | 3918 | 4296 | 5012 | 3492 |

A = gemäß der Erfindung
B — E = Vergleichsverbindungen

Tabelle III

Calcit-Inhibierung (einges. 4133 mg $CaCO_3$/l)

| | Inhibitor | 5 ppm | 10 ppm | 20 ppm | 40 ppm | Blindwert |
|---|---|---|---|---|---|---|
| | | Verbliebener Calciumcarbonatanteil in Lösung in mg/l | | | | |
| A | $Me_2N(CH_2)_3C(OH)(PO_3H_2)_2$ | 4020 | 4092 | 4140 | 4101 | 2360 |
| B | $H_2N(CH_2)_3C(OH)(PO_3H_2)_2$ | 2814 | 2966 | 3044 | 3408 | 2400 |
| C | $CH_3CH(NH_2)CH_2C(OH)(PO_3H_2)_2$ | 3222 | 3200 | 3412 | 3490 | 2375 |
| D | $H_2N(CH_2)_2C(OH)(PO_3H_2)_2$ | 2664 | 2612 | 2878 | 3010 | 2430 |
| E | $Me_2N(CH_2)_2C(OH)(PO_3H_2)_2$ | 3657 | 3692 | 3980 | 3998 | 2440 |

A = gemäß der Erfindung
B — E = Vergleichsverbindungen

Aus dem Vergleich der erfindungsgemäßen DABD gegenüber den Vergleichssubstanzen B bis E ist ersichtlich, daß DABD ein wesentlich besseres Komplexierungsvermögen aufweist und eine wesentlich erhöhte Threshold-Wirkung. Wie man beispielsweise aus Tabelle II ersieht, wird im Vergleich zum Blindwert bei Einsatz der erfindungsgemäßen DABD beispielsweise schon bei 1 ppm nahezu eine vollständige Inhibierung festgestellt, während bei den Vergleichssubstanzen eine merkbare Inhibierung erst bei 20 ppm und darüber auftritt. Ähnliche Ergebnisse lassen sich aus der Tabelle III entnehmen.

Daraus folgt, daß die erfindungsgemäße DABD in überraschender Weise ein besseres Komplexierungsvermögen und eine verbesserte Threshold-Aktivität aufweist als die bekannten ähnlichen und analogen Verbindungen. Dies war für den Fachmann in keiner Weise voraussehbar.

**Patentansprüche**

1. 4-Dimethylamino-1-hydroxybutan-1,1-diphosphonsäure der Formel

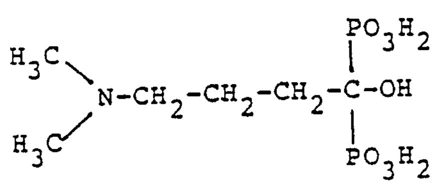

und deren wasserlösliche Salze.

2. Verwendung der Verbindung nach Anspruch 1 als Komplexiermittel.
3. Pharmazeutische Zusammensetzungen, enthaltend eine Verbindung nach Anspruch 1, zusammen mit üblichen pharmazeutischen Trägern und Hilfsstoffen.

**Claims**

1. 4-dimethylamino-1-hydroxybutane-1,1-diphosphonic acid corresponding to the following formula

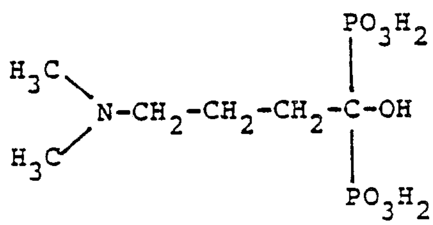

and water-soluble salts thereof.
2. The use of the compound claimed in Claim 1 as a complexing agent.
3. Pharmaceutical preparations containing a compound of the type claimed in Claim 1 together with standard pharmaceutical carriers and auxiliaries.

**Revendications**

1. Acide 4-diméthylamino-1-hydroxybutane-1,1-diphosphonique de formule :

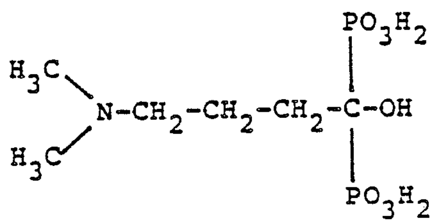

et ses sels solubles dans l'eau.
2. Utilisation du composé selon la revendication 1 en tant qu'agent complexant.
3. Compositions pharmaceutiques contenant un composé, selon la revendication 1 ensemble avec des supports pharmaceutiques usuels et des substances auxiliaires.